# EUROPEAN PATENT SPECIFICATION

(11) **EP 0 699 049 B1**
(45) Date of publication and mention of the grant of the patent: **07.04.1999**
(21) Application number: 94915622.8
(22) Date of filing: 19.05.1994
(51) Int. Cl.: A61B 5/0428, A61B 5/0404

(54) **MULTICHANNEL RECORDING APPARATUS**
MEHRKANALIGES AUFZEICHNUNGSGERÄT
APPAREIL D'ENREGISTREMENT A MULTICANAUX

(30) Priority: 19.05.1993 GB 9310359
(43) Date of publication of application: 06.03.1996
(73) Proprietor: NEILSON, James McEwan McIntyre, Edinburgh EH9 2EU (GB)
(72) Inventor: NEILSON, James McEwan McIntyre, Edinburgh EH9 2EU (GB)
(74) Representative: Cline, Roger Ledlie
(86) International application number: GB9401075
(87) International publication number: WO9426164

(56) References cited:
- WO-A-89/09021
- US-A- 4 367 752
- US-A- 4 622 979

## Description

Many types of electrical signal can be picked up from the living body by applying pairs of electrodes to the body at points appropriate to detect the signals of interest. For example, a pair of electrodes applied to the head will pick up the potential difference between the points chosen due to the electroencephalogram signal from the brain. Again, a pair of electrodes applied to the thorax can pick up an electrocardiogram (ecg) signal from the heart. These signals are of relatively very small voltage compared to the interference voltage picked up on the body as a whole relative to earth due to the proximity of the subject to main$ wiring, electrical apparatus, and other sources of electrical noise.

It used to be the practice to apply a third or 'earth' electrode to the subject's body to reduce the effects of this interference and more recently this third electrode is still used to connect unearthed amplifier devices to the subject's body as a reference connection to try to equalise the interference voltage picked up on the subject's body and on the unearthed amplifier in apparatus separate from the subject's body, such as a trolley-mounted ecg recording machine, and 'bedside' ecg patient monitoring apparatus.

WO-A-89 09021 relates to a portable body electrode monitor comprising three electrodes, three amplifiers each having two signal inputs, and data processing apparatus connected to the amplifiers, the sole connections between each amplifier and the electrodes being connections from the two signal inputs to two electrodes, different amplifiers being connected to different combinations of electrodes.

In ambulatory monitoring the complete electronic system including amplifiers, recording or telemetering apparatus and/or automatic signal analysis apparatus is miniaturised and battery operated, and is adapted for wearing on the subject's body, for example in a pouch often mounted on a waist belt. The whole apparatus is small enough and close enough to the subject so that its environment is the subject's body and it exists within his 'electrical shadow' as it were. Thus the subject and his ambulatory monitoring apparatus are subject to the same electrical noise fields and the interference creates relatively little difference in potential between the ambulatory monitoring circuit chassis and the subject's body.

The invention provides a body electrode monitor as defined in claim 1 and a method of monitoring a living body as defined in claim 5.

The characterising features of claim 1 or 5 mean that the monitor is suitable for ambulatory monitoring, where the whole equipment is carried by the body. There is no need for a reference connection to an additional body electrode in addition to the connection of the two inputs already mentioned. The former practice of using a reference electrode and lead in ambulatory monitoring has been carried over from the conventional 'ground base' monitoring into ambulatory monitoring practice quite unnecessarily.

In a preferred arrangement each electrode is connected to an input of a plurality of amplifiers, preferably by a single cable leading to a junction adjacent the amplifiers to keep the number of cables adjacent the electrodes (which might cause discomfort to the body) to a minimum.

An example of the invention will now be described with reference to the accompanying drawings in which
Figure 1 represents a prior art arrangement using three amplifiers, and
Figure 2 represents an arrangement using three amplifiers according to the present invention.

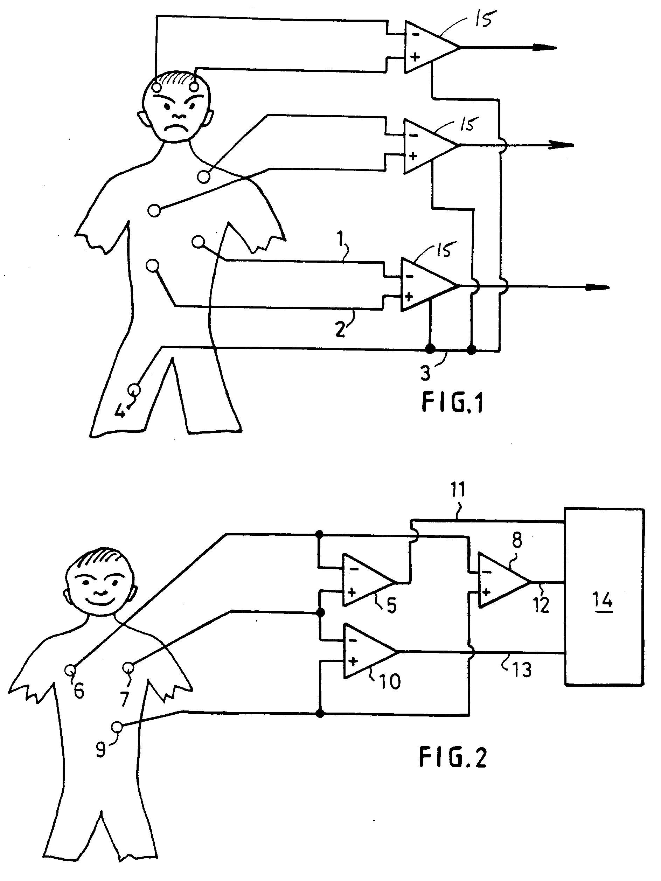

In Figure 1, each amplifier 15 has two inputs 1, 2 and each input is connected to its own body electrode. In addition, each amplifier is connected to an earth line 3 which is connected to a further separate body electrode 4. Thus there are three connections between each amplifier and the electrodes. It will be seen that for three amplifiers seven body electrodes are required and in the general case, n amplifiers require (2n + 1) body electrodes. One amplifier is required for each body parameter being measured and so for a greater number of body parameters, the number of body electrodes and their individual connecting cables becomes very large and is a cause of discomfort to the subject.

In Figure 2, there are two basic differences over Figure 1. Firstly, there is no reference connection between each amplifier and a further reference body electrode. The other difference is that there are only two connections between each amplifier and the electrodes. The figure 2 arrangement contains the optional feature that each body electrode is connected to two inputs of differential amplifiers, one input from one amplifier, and another input from a different amplifier so that different combinations of only two electrodes are connected to different amplifiers. The amplifiers are differential amplifiers which have the advantage that the two active input connections of such an amplifier would have 'balanced', i.e. approximately equal, and relatively high impedances between the input terminals and the 'floating' common or chassis connection of the amplifier. This would overcome the effect of any remaining small difference of potential between the body and the bulk of the amplifier circuit (i.e. its 'common' or chassis connection).

The combined result of these differences is that the number of body electrodes and the corresponding number of connecting cables is reduced from seven to three. In the general case, if n electrodes are applied to the body connected in pairs to separate patient worn amplifiers so that each amplifier connects to only two of the electrodes but individual electrodes may be shared between two or among more than two amplifiers then n electrodes can service nC2 = 0.5n(n-1) amplifiers. Ignoring the fact that some parameters may not be useful, this means 0.5n(n-1) different body parameters can be measured using n body electrodes and n connecting cables. The seven electrodes of Figure 1 could service 21 channels using the differential connecting arrangement analogous to that of Figure 2.

In Figure 2, it will be seen that the first body electrode 6 is connected to the negative input of differential amplifier 5 and the negative input of differential amplifier 8. The body electrode 7 is connected to the positive input of differential amplifier 5 and the negative input of differential amplifier 10. The third body electrode is connected to the positive input of differential amplifier 10 and the positive input of differential amplifier 8. The outputs 11, 12 and 13 of differential amplifiers 5, 8 and 10 respectively are connected to data processing apparatus 14 which may simply be a recorder or a display or may combine these items alone or together with data processing apparatus in order to interpret the difference of potential arising at the pairs of body electrodes in terms of body parameters to be measured.

The differential amplifiers, which are adapted to be carried by the subject, are preferably carried in a belt mounted pouch. The data processing apparatus can be similarly mounted, preferably in a casing for the amplifiers and apparatus.

## Claims

1. A body electrode monitor comprising a plurality of electrodes (6,7,9) for sensing potentials at points of attachment on a living body, a plurality of amplifiers (5,8,10) each having two signal inputs, and data processing apparatus (14) connected to the amplifiers, the sole connections between each amplifier and the electrodes being connections from the two signal inputs to two electrodes, different amplifiers being connected to different combinations of electrodes, characterised in that a casing for the amplifiers (5,8,10) and the data processing apparatus (14) is provided with belt attachment means, and the amplifiers and data processing apparatus are small enough and close enough to the body so that their environment is the subject's body such that the amplifiers and the data processing apparatus are subject to the same electrical noise fields as the body and they exist within the subject's electrical shadow.

2. A monitor as claimed in claim 1 wherein one of said plurality of electrodes (6,7,9) is connected to an input of a plurality of said amplifiers (5,8,10).

3. A monitor as claimed in claim 2 wherein each of said plurality of electrodes (6,7,9) is connected to an input of a plurality of said amplifiers (5,8,10).

4. A monitor as claimed in any one of claims 1 to 3 wherein the amplifiers are differential amplifiers (5,8,10).

5. A method of monitoring a living body, the method comprising providing a plurality of electrodes (6, 7, 9) for sensing potentials at points of attachment on said body, providing a plurality of amplifiers (5,8,10), each having two signal inputs, and providing data processing apparatus (14) connected to the amplifiers, the sole connections between each amplifier and the electrodes being connections from the two signal inputs to two electrodes, different amplifiers being connected to different combinations of electrodes, characterised in that the amplifiers (5,8,10) and the data processing apparatus (14) are in a casing which is carried by the body and are small enough and close enough to the body so that their environment is the subject's body such that the amplifiers and the data processing apparatus are subject to the same electrical noise fields as the body and they exist within the subject's electrical shadow.

6. A method as claimed in claim 5, wherein one of said plurality of electrodes (6, 7, 9) is connected to an input of a plurality of said amplifiers (5,8,10).

7. A method as claimed in claim 6, wherein each of said plurality of electrodes (6, 7, 9) is connected to an input of a plurality of said amplifiers (5,8,10).

8. A method according to claims 5, 6 or 7, wherein the amplifiers (5,8,10) and the data processing apparatus (14) are attached to a belt which is worn on the body.

## Patentansprüche

1. Körperelektroden-Überwachungsgerät, aufweisend mehrere Elektroden (6, 7, 9) zum Erfühlen von Potentialen an Befestigungspunkten an einem lebenden Körper, mehrere Verstärker (5, 8, 10), die jeweils zwei Signaleingänge aufweisen, und eine Datenverarbeitungsvorrichtung (14), die mit den Verstärkern verbunden ist, wobei es sich bei den einzigen Verbindungen zwischen jedem Verstärker und den Elektroden um Verbindungen von den zwei Signaleingängen zu zwei Elektroden handelt, wobei unterschiedliche Verstärker mit unterschiedlichen Kombinationen von Elektroden verbunden sind, dadurch gekennzeichnet, daß ein Gehäuse für die Verstärker (5, 8, 10) und die Datenverarbeitungsvorrichtung (14) mit Riemenbefestigungseinrichtungen versehen sind, und die Verstärker und die Datenverarbeitungsvorrichtung klein genug und ausreichend nahe am Körper liegend derart sind, daß ihre Umgebung der Körper des zu Untersuchenden derart ist, daß die Verstärker und die Datenverarbeitungsvorrichtung denselben elektrischen Rauschfeldern ausgesetzt sind wie der Körper und sie innerhalb des elektrischen Schaffens des Untersuchten liegen.

2. Überwachungsgerät nach Anspruch 1, wobei eine der mehreren Elektroden (6, 7, 9) mit einem Eingang von mehreren der Verstärker (5, 8, 10 ) verbunden ist.

3. Überwachungsgerät nach Anspruch 2, wobei jede der mehreren Elektroden (6, 7, 9) mit einem Eingang von mehreren der Verstärker (5, 8, 10) verbunden ist.

4. Überwachungsgerät nach einem der Ansprüche 1 bis 3, wobei es sich bei den Verstärkern um Differenzverstärker (5, 8, 10) handelt.

5. Verfahren zum Überwachen eines lebenden Körpers, wobei das Verfahren vorsieht: Bereitstellen von mehreren Elektroden (6, 7, 9) zum Erfühlen von Potentialen an Befestigungspunkten auf dem Körper, Bereitstellen von mehreren Verstärkern (5, 8, 10), von denen jeder zwei Signaleingänge aufweist, und Bereitstellen einer Datenverarbeitungsvorrichtung (14), die mit den Verstärkern verbunden ist, wobei die einzigen Verbindungen zwischen jedem Verstärker und den Elektroden Verbindungen von den zwei Signaleingängen zu zwei Elektroden sind, wobei unterschiedliche Verstärker mit unterschiedlichen Kombinationen von Elektroden verbunden sind, dadurch gekennzeichnet, daß die Verstärker (5, 8, 10 ) und die Datenverarbeitungsvorrichtung (14) in einem Gehäuse angeordnet sind, das durch den Körper getragen wird, und daß sie klein genug und ausreichend nahe zum Körper liegend so sind, so daß ihre Umgebung der Körper des zu Untersuchenden ist, so daR die Verstärker und die Datenverarbeitungsvorrichtung denselben elektrischen Rauschfeldern wie der Körper unterworfen sind und sie in dem elektrischen Schatten des zu Untersuchenden vorliegen.

6. Verfahren nach Anspruch 5, wobei eine der mehreren Elektroden (6, 7, 9) mit einem Eingang von mehreren der Verstärker (5, 8, 10 ) verbunden ist.

7. Verfahren nach Anspruch 6, wobei jede der mehreren Elektroden (6, 7, 9) mit einem Eingang von mehreren der Verstärker (5, 8, 10 ) verbunden ist.

8. Verfahren nach Anspruch 5, 6 oder 7, wobei die Verstärker (5, 8, 10 ) und die Datenverarbeitungsvorrichtung (14) an einem Riemen befestigt sind, der auf dem Körper getragen wird.

## Revendications

1. Moniteur à électrodes conçu pour le corps humain comprenant une pluralité d'électrodes (6, 7, 9) permettant de détecter des potentiels au niveau de points de fixation sur un corps humain, une pluralité d'amplificateurs (5, 8, 10), chacun ayant deux entrées de signal et un appareil de traitement de données (14) connecté aux amplificateurs, les connexions uniques entre chaque amplificateur et les électrodes étant des connexions allant de deux entrées de signal à deux électrodes, des amplificateurs différents étant connectés à des combinaisons différentes d'électrodes, caractérisé en ce qu'un boîtier destiné aux amplificateurs (5, 8, 10) et à l'appareil de traitement de données (14) est prévu avec un moyen de fixation par ceinture et en ce que les amplificateurs et l'appareil de traitement de données sont suffisamment petits et suffisamment proches du corps de sorte que leur environnement est le corps du sujet de sorte que les amplificateurs et l'appareil de traitement de données sont soumis aux mêmes champs de parasites électriques que le corps et qu'ils existent dans l'ombre électrique du sujet.

2. Moniteur suivant la revendication 1, dans lequel une électrode de ladite pluralité d'électrodes (6, 7, 9) est connectée à une entrée d'une pluralité desdits amplificateurs (5, 8, 10).

3. Moniteur suivant la revendication 2, dans lequel chacune de ladite pluralité d'électrodes (6, 7, 9) est connectée à une entrée d'une pluralité desdits amplificateurs (5, 8, 10).

4. Moniteur suivant l'une quelconque des revendications 1 à 3, dans lequel les amplificateurs sont des amplificateurs différentiels (5, 8, 10).

5. Procédé de surveillance d'un corps humain, le procédé comprenant les mesures consistant à pourvoir à une pluralité d'électrodes (6, 7, 9) permettant de détecter des potentiels au niveau de points de fixation sur ledit corps, à pourvoir à une pluralité d'amplificateurs (5, 8, 10), chacun comportant deux entrées de signal, ainsi qu'à pourvoir à un appareil de traitement de données (14) connecté aux amplificateurs, les connexions uniques entre chaque amplificateur et les électrodes étant des connexions allant de deux entrées de signal à deux électrodes, des amplificateurs différents étant connectés à des combinaisons différentes d'électrodes, caractérisé en ce que les amplificateurs (5, 8, 10) et l'appareil de traitement de données (14) sont dans un boîtier qui est transporté par le corps et sont suffisamment petits et suffisamment proches du corps, de sorte que leur environnement est le corps du sujet de telle manière que les amplificateurs et l'appareil de traitement de données sont soumis aux mêmes champs de parasites électriques que le corps et qu'ils existent dans l'ombre électrique du sujet.

6. Procédé suivant la revendication 5, dans lequel l'une de ladite pluralité d'électrodes (6, 7, 9) est connectée à l'entrée d'une pluralité desdits amplificateurs (5, 8, 10).

7. Procédé suivant la revendication 6, dans lequel chacune de ladite pluralité d'électrodes (6, 7, 9) est connectée à l'entrée d'une pluralité desdits amplificateurs (5. 8, 10).

8. Procédé suivant les revendication 5, 6 ou 7, dans lequel les amplificateurs (5, 8, 10) et l'appareil de traitement de données (14) sont attachés à une ceinture qui est portée sur le corps.
